# FASCICULE DE BREVET EUROPEEN

(11) **EP 0 559 678 B1**
(45) Date de publication et mention de la délivrance du brevet: **11.01.1995**
(21) Numéro de dépôt: 91920106.1
(22) Date de dépôt: 28.11.1991
(51) Int. Cl.: C12M 1/24

(54) **APPAREIL POUR DEVELOPPER UNE SURFACE D'ECHANGE ROTATIVE**
VORRICHTUNG ZUM ENTWICKELN EINER ROTIERENDEN AUSTAUSCHFLÄCHE
APPARATUS FOR FORMING A REVOLVING EXCHANGE SURFACE

(30) Priorité: 29.11.1990 BE 9001137
(43) Date de publication de la demande: 15.09.1993
(73) Titulaire: SPIELMANN, Richard, B-7850 Enghien (BE)
(72) Inventeur: SPIELMANN, Richard, B-7850 Enghien (BE)
(74) Mandataire: Vanderperre, Robert
(86) Numéro de dépôt international: BE9100084
(87) Numéro de publication internationale: WO9209681

(56) Documents cités:
- WO-A-88/00235
- FR-A- 2 354 554
- GB-A- 2 055 397
- LU-A- 51 646
- US-A- 4 912 058

## Description

L'invention concerne un appareil pour développer une surface d'échange réactionnel régulièrement renouvelée entre une phase liquide et une phase gazeuse. Un appareil de ce genre est prévu, par exemple, pour la culture cellulaire et la production de substances biologiques.

Dans les industries produisant des vaccins par culture cellulaire ou produisant des substances biologiques sécrétées soit par des cellules adhérentes ou en suspension, soit par des micro-organismes, on utilise des microporteurs, des flacons roulants, des flacons de Roux, des systèmes à chambres multiples et des fermenteurs.

Les microporteurs sont des petites billes de taille microscopique sur lesquelles croissent des cellules adhérentes. Les billes sont maintenues en suspension dans le milieu de culture, appelé ci-après la phase liquide, à l'aide d'un système d'agitation, la phase liquide étant en contact avec une phase gazeuse. Le tout se trouve dans une cuve appelée fermenteur. Cette technique couramment utilisée dans l'industrie du vaccin est cependant confrontée à deux difficultés majeures :
- la difficulté d'effectuer une mise en suspension des microporteurs dans le milieu de culture compatible avec les conditions d'accrochage et de croissance cellulaire,
- la difficulté d'assurer le contrôle et la stabilité du pH du milieu de culture, en tenant compte du fait que la surface d'échange entre la phase gazeuse et la phase liquide est nettement limitée.

Les fermenteurs sont également utilisés pour la culture de cellules en suspension ou de micro-organismes. Les difficultés d'utilisation sont similaires à celles rencontrées lors de la culture avec microporteur; la surface d'échange entre la phase gazeuse et liquide étant limitée par le diamètre du fermenteur, la stabilité du pH devient extrêmement difficile à assurer.

Les flacons roulants sont généralement de forme cylindrique et sont conçus pour tourner sur leurs axes. Les surfaces intérieures de ces flacons sont prévues pour cultiver des cellules adhérentes formant ainsi des surfaces de culture. Le liquide de culture est introduit avec les cellules à l'intérieur du flacon. Le mouvement de rotation auquel sont soumis les flacons permet de recouvrir d'un film de milieu les surfaces de culture, permettant ainsi la croissance cellulaire sur ces surfaces de culture. La surface de culture est donc limitée à la taille des flacons. Si on veut produire une grande quantité de cellules, un grand nombre de flacons sera nécessaire. Ceci est le cas dans les industries produisant par exemple de l'interferon, de l'insuline, des vaccins viraux, des lymphokines. La manipulation de ces nombreux flacons lors de l'innoculation, du changement de milieu, de l'introduction du virus, de la récolte du surnageant et des cellules, augmente le risque de contamination des flacons et de leur contenu et nécessite l'utilisation d'un personnel important. Plusieurs appareils ont été mis au point de manière à augmenter la surface de culture des flacons roulants en augmentant la surface du flacon lui-même. Ces appareils connus sont destinés à effectuer des cultures de cellules adhérentes mais ne sont pas prévus pour assurer une mise en suspension homogène des microporteurs ou des cellules en suspension.

Les flacons de Roux sont utilisés depuis plus d'un siècle pour produire des virus, ainsi que d'autres substances biologiques. Ils présentent les mêmes inconvénients que les flacons roulants, c'est-à-dire une surface limitée de culture par flacon et ils requièrent un personnel de manutention important.

Le document EP-A-0345415 divulgue un appareil dans lequel le flacon présente une surface agrandie. Cette augmentation de surface est obtenue en conférant au corps du flacon une surface ondulée axialement ou longitudinalement par rapport à l'axe du flacon. Ce flacon ne permet cependant pas de brassage des liquides. Ainsi il ne permet pas de culture avec microporteurs.

La surface utile pour le développement des cellules est également augmentée avec l'appareil divulgué par le document US-A-3.839.155. Dans cet appareil, la surface additionnelle est obtenue par l'agencement parallèle d'une série de plateaux sur l'axe du flacon. Cet appareil n'est toutefois pas entièrement satisfaisant pour les cellules adhérentes car les plateaux, lors-qu'ils sont en position horizontale, ne peuvent retenir un volume de liquide contenant la suspension cellulaire. Cela a pour conséquence que le liquide s'écoule du plateau, ce qui ne favorise pas l'accrochage cellulaire lors de la mise en culture. De plus, cet appareil n'est pas approprié pour la culture de cellules dites non adhérentes car rien n'est prévu ici pour le brassage de la phase liquide.

Le document LU-A-51646, quant à lui, décrit un appareil de culture de cellules comprenant un ensemble de plateaux parallèles placés l'un au-dessus de l'autre de manière à former des chambres de culture. Le tout est placé à l'intérieur d'une enceinte qui peut basculer autour d'un axe. Ce système permet de cultiver des cellules adhérentes sur une seule face des plateaux seulement. La surface intérieure de l'enceinte n'est pas utilisée comme surface de culture. De plus, on ne peut y introduire un système de brassage permettant de maintenir des microporteurs ou des micro-organismes en suspension homogène et continue.

La présente invention a pour but de remédier à ces inconvénients. A cette fin, elle propose un appareil comprenant une enceinte fermée allongée délimitant un volume pour recevoir un liquide de réaction, un dispositif pour introduire le liquide de réaction dans l'enceinte et pour l'en évacuer, et au moins un ensemble de plateaux disposés longitudinalement à l'intérieur de l'enceinte avec un écartement régulier entre eux.

Les plateaux présentent le long de leurs bords, des rebords qui font saillie sur au moins une des faces opposées des plateaux de manière que, lorsque l'on imprime un mouvement de rotation à l'ensemble de plateaux précité, les plateaux plongent successivement dans le liquide de réaction et retiennent chacun une quantité prédéterminée de liquide pendant une fraction de leur course angulaire.

Ainsi, grâce à l'agencement des plateaux proposée, suivant l'invention, la surface d'échange ou de culture se trouve-t-elle considérablement accrue à volume égal et une mise en suspension de cellules non adhérentes, de micro-organismes, de microporteurs ou de microsphères est-elle assurée sans devoir utiliser un système d'agitation interne. La rotation des plateaux assure un excellent brassage de la phase liquide introduite dans l'appareil grâce à l'entraînement du liquide par les plateaux en rotation. Ce brassage et les mouvements du liquide à l'intérieur de l'enceinte obtenus grâce à la rotation des plateaux assurent une répartition homogène des éléments de la phase liquide et accroissent fortement l'efficacité de l'échange et de la culture.

Les plateaux peuvent être disposés et fixés de diverses manières. Dans un mode de réalisation exemplaire, les plateaux sont disposés radialement autour d'un axe s'étendant longitudinalement dans l'enceinte et se trouvent fixés sur ledit axe ou sur la surface intérieure de l' enceinte ou encore à la fois sur l'axe et sur la surface intérieure de l' enceinte. Les plateaux sont avantageusement amovibles se glissant par exemple dans des rainures prévues pour recevoir leurs bords longitudinaux. Les faces des plateaux peuvent être formées avec des ondulations, ce qui accroît encore le développement et le rendement de la surface d'échange ou de culture.

Un autre mode de réalisation intéressant car il est relativement simple de construction, comprend un ensemble de plateaux parallèles assemblés pour présenter un écartement régulier entre eux. Dans ce mode d'exécution aussi, les plateaux peuvent être fixés entre eux pour former un ensemble rotatif à l'intérieur de l'enceinte ou être montés sur la paroi de l'enceinte, par exemple par glissement dans des rainures prévues dans la surface intérieure de l'enceinte.

Divers autres détails de réalisation de l'appareil conforme à l'invention sont définis dans les sous-revendications.

De manière avantageuse, l'invention prévoit de faire tourner les plateaux alternativement d'un demi-tour au moins dans un sens puis dans l'autre de manière à obtenir un excellent mouillage des faces opposées des plateaux par un film d'agent de réaction.

L'invention est décrite plus en détail ci-après, à titre d'exemple, à l'aide des dessins annexés représentant quelques modes d'exécution préférés.
La figure 1 représente une vue éclatée en perspective, partiellement en écorché, d'un premier mode de réalisation conforme à la présente invention, dans lequel les plateaux sont agencés radialement.
La figure 2 représente, à échelle agrandie, une vue en coupe transversale suivant la ligne II-II de la figure 1.
La figure 3 est une vue en coupe transversale d'un deuxième mode de réalisation exemplaire selon l'invention.
La figure 4 est une vue partielle, en coupe transversale, d'un troisième mode de réalisation exemplaire selon l'invention.
La figure 5 est une vue en perspective d'un plateau exemplaire de l'appareil selon la figure 4.
La figure 6 représente une vue en coupe transversale d'un quatrième mode de réalisation exemplaire selon l'invention, dans lequel les plateaux sont agencés parallèlement les uns aux autres.
La figure 7 est une vue en coupe longitudinale de l'appareil selon la figure 6.

Dans les dessins, des signes de référence identiques désignent des organes ou éléments identiques ou semblables et équivalents.

D'une manière générale, un appareil suivant la présente invention comprend une enceinte fermée allongée qui délimite un volume pour recevoir un liquide de réaction et un ensemble de plateaux disposés longitudinalement à l'intérieur de l'enceinte avec un écartement régulier entre eux. L'ensemble des plateaux est monté à rotation de manière à pouvoir tourner autour d'un axe longitudinal grâce à des moyens d'entraînement quelconques, connus en soi. Le long de leurs bords, les plateaux sont pourvus de rebords qui font saillie sur une face ou sur les deux faces de chacun des plateaux de manière à agir comme élément de retenue pour du liquide de réaction dans lequel les plateaux sont amenés à plonger tour à tour pendant leur rotation.

Dans ce qui suit on décrira à titre d'exemple particulier un appareil appelé bioréacteur rotatif destiné à permettre la culture de cellules adhérentes ou non adhérentes avec ou sans microporteurs ou la culture de bactéries et autres micro-organismes. A cette fin, s'il s'agit de culture de cellules adhérentes par exemple, dans l'enceinte du bioréacteur est introduit un liquide destiné à faire naître et croître les cellules et micro-organismes sur les faces opposées des plateaux et sur les parois intérieures de l'enceinte. Pour améliorer les performances de culture, il faut augmenter la surface disponible susceptible d'entrer en contact avec le liquide de réaction et la rendre également exploitable au mieux.

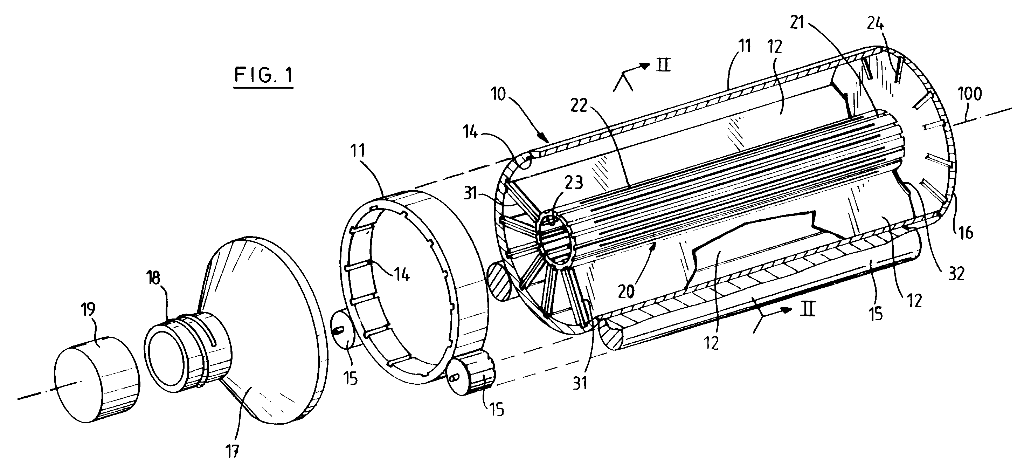

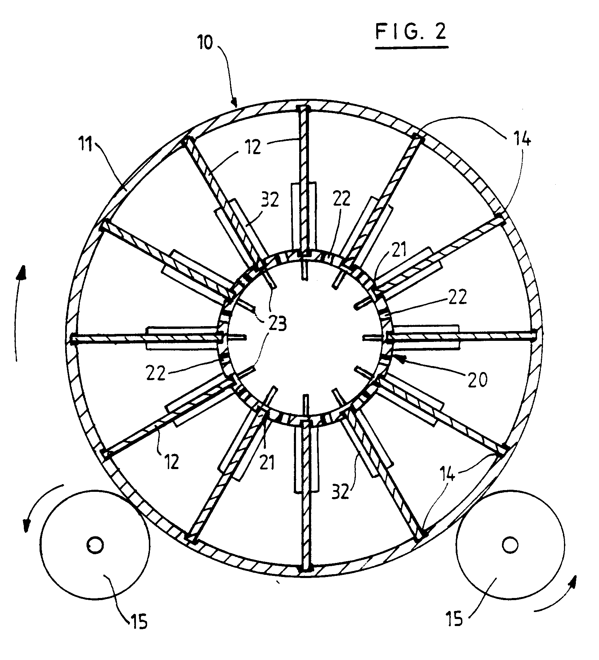

L'exemple de réalisation illustré aux figures 1 et 2 montre un bioréacteur rotatif 10 en forme de flacon comprenant une enceinte extérieure 11 de forme cylindrique, montée sur des rouleaux 15 pour être entraînée en rotation autour de son axe longitudinal 100. A ses extrémités opposées, l'enceinte 11 présente un fond 16 et une encolure 17. Celle-ci est munie d'un col 18 concentrique à l'axe longitudinal 100 de l'enceinte et servant à recevoir un organe de fermeture tel qu'un capuchon 19 à vis. A l'intérieur de l'enceinte, les plateaux 12 sont disposés radialement autour d'un axe 20 s'étendant longitudinalement dans l'enceinte avec un écartement régulier entre eux. Les plateaux 12 s'étendent sur pratiquement toute la longueur de l'enceinte jusqu'à une distance prédéterminée du fond 16 et de l'encolure 17 précités. Les plateaux 12 peuvent toutefois également être de longueur plus courte. Ces plateaux 12, qui présentent chacun pratiquement une forme rectangulaire, sont normalement rigides de telle sorte qu'ils puissent supporter leur propre poids ainsi que celui du liquide qu'ils sont destinés à retenir ainsi qu'on le verra plus loin. Leur nombre peut varier en fonction des caractéristiques techniques de l'appareil.

Dans le mode d'exécution illustré sur les figures 1 et 2, les plateaux s'étendent depuis l'axe 20 jusqu'à l'enceinte 11 et ils sont fixés par leurs deux côtés opposés, respectivement à l'axe 20 et à l'enceinte 11. A cette fin, la surface intérieure de l'enceinte 11 et la surface extérieure de l'axe 20 présentent des rainures longitudinales 14 et 21 respectivement, agencées de manière à assurer une fixation sûre des plateaux. Les rainures 14 et 21 précitées s'étendent sur pratiquement toute la longueur de l'enceinte 11 et de l'axe 20.

Les bords latéraux 28 et 29 de chaque plateau 12 sont pourvus de rebords 31 et 32 faisant saillie sur une face ou sur les deux faces opposées du plateau. Une partie du bord latéral 29 destiné à se trouver au fond de l'enceinte 11 est prévue pour s'appuyer sur le fond 16 et s'engager dans des rainures radiales 24 prévues dans la face intérieure du fond 16. L'autre partie du bord latéral 29 présente une courbure et est pourvue d'un rebord 32 faisant saillie sur le plan du plateau. Ladite courbure permet d'assurer un alignement entre le plateau et l'extrémité adjacente de l'axe 20 lors de leur fixation et ménage un passage pour les gaz. Les rebords précités peuvent éventuellement avoir une hauteur qui décroît depuis le bord longitudinal 26. Chaque plateau avec les rebords précités forment pratiquement une cuvette permettant de retenir momentanément une quantité déterminée du liquide de réaction contenu dans l'enceinte lorsque chaque plateau plonge à son tour dans le liquide de réaction durant la rotation de l'ensemble et ce pendant une fraction de sa course angulaire ascendante.

L'axe 20 est avantageusement creux et présente des ouvertures longitudinales 22 parallèles qui alternent avec les rainures 21. Les ouvertures 22 permettent l'écoulement du liquide de réaction à l'intérieur de l'axe 20. Lors de la rotation des plateaux, le liquide recouvre alors des nervures 23 disposées sur la surface intérieure de l'axe 20. Le liquide de réaction s'écoule ensuite sur les nervures 23 situées en contrebas, traverse les ouvertures 22 correspondantes et s'écoule le long des plateaux 12 en position inférieure. Il va de soi que l'axe 20 pourrait tout aussi bien être plein au lieu d'être creux.

Les plateaux présentent une surface généralement plane. Cependant ils peuvent aussi présenter une forme légèrement gauchie. Dans le but d'accroître la surface totale de culture de chaque plateau, les faces des plateaux présentent des ondulations, par exemple sous forme de striures. Ceci augmente encore le développement de la surface de réaction ou d'échange et rend la culture de cellules ou micro-organismes très efficace.

La surface intérieure de l'enceinte 11 ainsi que chacune des faces opposées 24 et 25 des plateaux 12 sont traitées pour la culture cellulaire soit avant assemblage, soit lors de l'assemblage partiel ou soit encore quand l'appareil est complètement assemblé et cela dans le but d'obtenir un traitement de surface optimal compatible avec la nature de la substance ou du micro-organisme que l'on veut y faire adhérer ou y cultiver.

Lors de l'assemblage de l'appareil, avant de fixer le fond 16 hermétiquement à l'enceinte 11, les plateaux 12 et l'axe 20 sont glissés à l'intérieur de l'enceinte 11 de manière que les plateaux 12 soient insérés dans les rainures 21 de l'axe 20 et dans les rainures radiales 24 du fond 16. Ce qui précède ne décrit qu'une manière d'assembler l'appareil. Les plateaux peuvent également être glissés par l'encolure 17 dans leurs rainures respectives 14, 21, 24 situées sur l'enceinte 11, sur l'axe 20 et sur le fond 16. Ici c'est l'encolure 17 qui est fixée hermétiquement pour fermer l'enceinte. Lorsque les plateaux 12 ont été introduits à l'intérieur de l'enceinte 11, celle-ci est alors fermée en fixant l'encolure 17.

L'agent de réaction et les substances à cultiver sont introduits par le col 18 de l'enceinte placée, par exemple, verticalement grâce à un dispositif pour introduire l'agent de réaction, connu en soi. Le remplissage de l'enceinte peut également se faire lorsque celle-ci est placée horizontalement. Dans ce cas, ce sont un ou des tuyaux (non montrés dans les figures) placés dans le col et se prolongeant soit dans l'axe de l'enceinte 11, soit le long de la surface intérieure de l'encolure 17 qui permet d'introduire ou de retirer des liquides, des gaz ou encore de vidanger l'enceinte de son contenu sans devoir l'ouvrir.

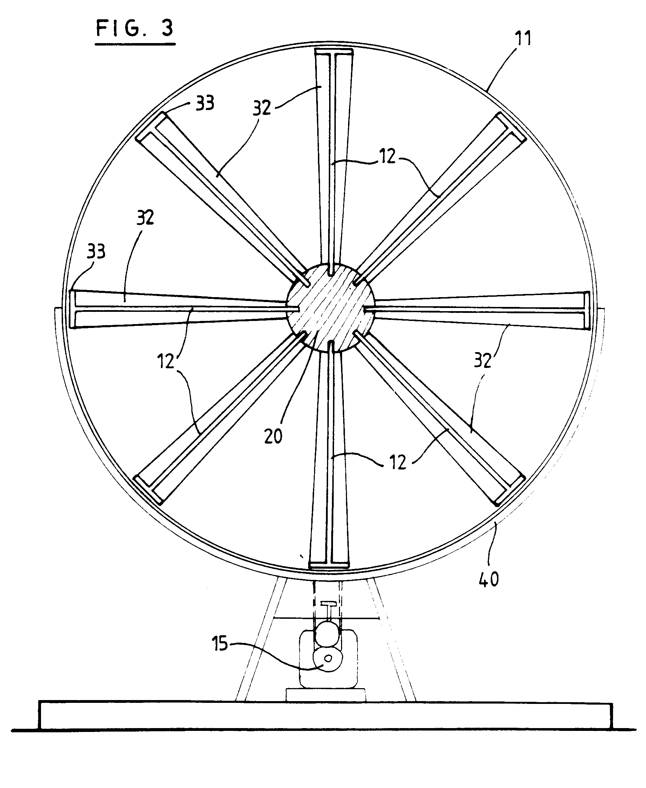

Dans l'exemple décrit plus haut, les plateaux 12 sont fixés par leurs deux bords longitudinaux. Il est à noter que les plateaux 12 peuvent n'être fixés que par l'un de leurs bords longitudinaux, soit qu'ils soient fixés par un bord sur l'axe 20 uniquement, soit qu'ils soient fixés par un bord à l'enceinte 11 uniquement. Dans le premier cas l'axe 20 et les plateaux 12 qu'il porte forment un ensemble monté de manière à être entraîné en rotation à l'intérieur de l'enceinte. La figure 3 représente un exemple de réalisation dans lequel les plateaux 12 se trouvent fixés sur l'axe 20 qui est ici un axe plein. Les plateaux sont calés dans des rainures formées longitudinalement dans la surface extérieure de l'axe. Celui-ci est monté à l'intérieur d'une enceinte 11 supportée par un bâti 40. Le bord longitudinal libre de chaque plateau 12 est muni d'un rebord longitudinal 33 qui fait saillie sur les faces du plateau. Le plateau avec le rebord longitudinal 33 et les rebords latéraux précités forme une cuvette propre à retenir une quantité de liquide de réaction comme expliqué plus haut. L'axe 20 est couplé par un dispositif de transmission à un moteur d'entraînement 15 de manière à faire tourner l'ensemble de plateaux autour de l'axe longitudinal de l'enceinte. Celle-ci pourrait même loger plusieurs ensembles de plateaux rotatifs tournant autour d'axes parallèles.

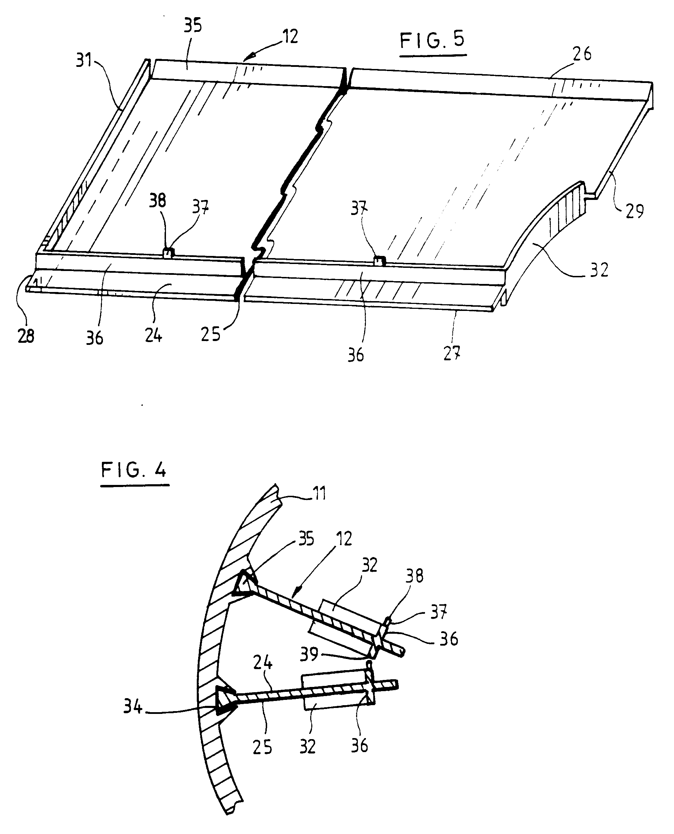

Dans le cas où les plateaux sont fixés uniquement à l'enceinte, c'est celle-ci qui entraîne les plateaux en rotation autour de l'axe longitudinal de l'enceinte. La figure 4 illustre un exemple de mode de réalisation dans lequel les plateaux sont fixés sur l'enceinte. Les plateaux 12 sont fixés à l'aide de languettes 34 mortaisées en forme de queue d'aronde permettant d'y faire coulisser le tenon en forme de queue d'aronde 35 formé sur le bord longitudinal extérieur 26 du plateau 12. Ce mode de fixation des plateaux s'avère très solide et particulièrement approprié lorsqu' il y a lieu d'entraîner des masses importantes de liquide tout en préservant une interchangeabilité aisée des plateaux, laquelle peut s'avérer utile pour leur renouvellement en cas d'altération du plateau.

Sur ses faces opposées 24 et 25, chaque plateau 12 porte ici une cloison 36 placée longitudinalement le long du bord longitudinal libre 27 du plateau, le cas échéant sur toute la longueur du plateau 12. La figure 5 montre une vue en perspective d'un plateau ainsi aménagé. La cloison longitudinale 36 et les rebords latéraux 31 et 32 mentionnés précédemment forment avec le plateau pratiquement une cuvette capable de retenir momentanément une quantité déterminée de liquide de réaction pendant la course angulaire ascendante du plateau. Afin d'éviter que les bords libres des plateaux voisins ne se touchent si d'aventure les plateaux fléchissaient sous le poids du liquide retenu ou sous l'effet de vibrations, les cloisons longitudinales 36 portent sur leur bord au moins une protubérance 37 servant d'organe distanceur et offrant une surface d'appui 38 pour le bord 39 de la cloison 36 adjacente située sur le plateau 12 immédiatement voisin, de manière à assurer ainsi toujours un espacement entre deux plateaux immédiatement voisins et permettre le libre passage des gaz et le déversement des liquides pendant la rotation des plateaux.

Les bords des plateaux peuvent également présenter d'autres formes comme par exemple au moins un bord simple sans rebord perpendiculaire, un bord avec bourrelet ou encore bord du côté du fond s'appuyant entièrement sur le fond 16 de l'enceinte 11. Les rebords latéraux et les cloisons longitudinales présentent avantageusement la même hauteur dans la zone où ils se rejoignent. Les rebords latéraux peuvent éventuellement avoir une hauteur décroissant régulièrement à partir du bord longitudinal 26 vers le bord longitudinal 27 du plateau.

Lorsqu'une quantité appropriée de liquide et de gaz est introduite dans l'enceinte 11, celle-ci et les plateaux 12 ou les plateaux 12 seulement sont mis en rotation autour de l'axe longitudinal de l'enceinte grâce aux moyens d'entraînement 15 prévus à cet effet. Pendant leur rotation, les plateaux 12 plongent tour à tour dans le liquide de réaction et durant la course ascendante des plateaux, une quantité déterminée de liquide de réaction est retenue par les rebords de chaque plateau et entraînée par les plateaux. L'excédent de liquide se déverse au fur et à mesure par dessus les rebords et ensuite par les ouvertures 22 dans l'axe 20 ou, le cas échéant, par l'espacement entre deux cloisons adjacentes 36 de plateaux immédiatement voisins (dans le cas de l'exécution de la figure 4). Le liquide retombe dans la partie inférieure de l'enceinte en recouvrant toutes les surfaces situées en contrebas.

Une rotation d'au moins 180° dans un sens, suivie d'une rotation d'au moins 180° en sens inverse après retour au point de départ, permet de "mouiller" d'un film d'agent de réaction les faces opposées 24, 25 des plateaux 12 et les surfaces intérieures de l'enceinte et de cultiver des cellules adhérentes sur ces surfaces.

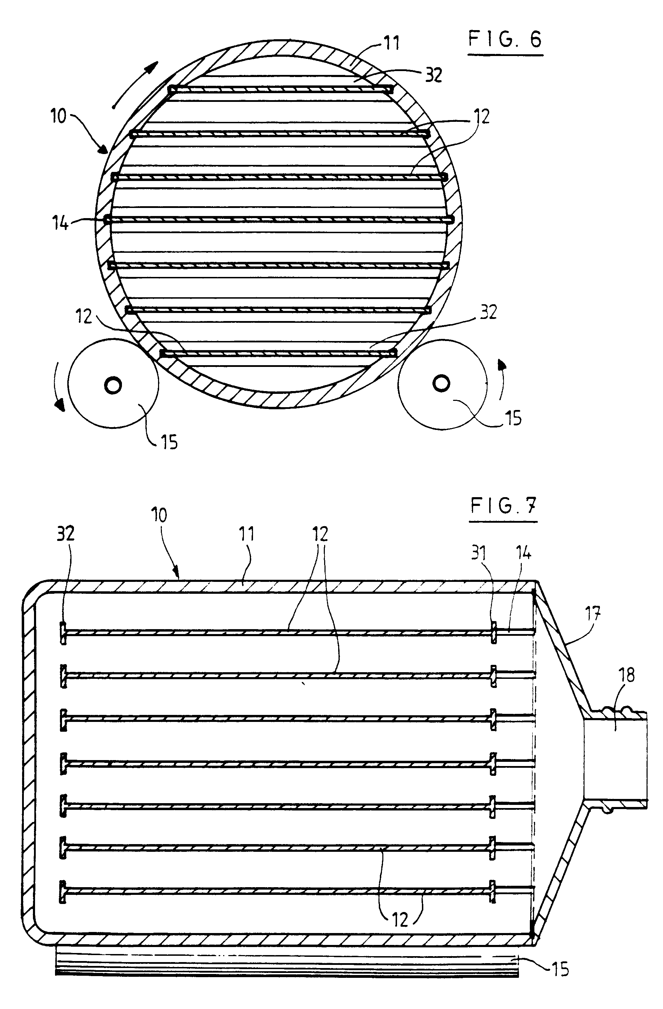

Les figures 6 et 7 illustrent un autre type d'agencement des plateaux. Ceux-ci sont montés parallèlement entre eux suivant la direction de l'axe longitudinal de l'enceinte et ils sont fixés à l'enceinte 11 par leurs deux bords longitudinaux opposés. Les plateaux peuvent également être fixés à l'enceinte 11 par un bord longitudinal seulement ou être fixés avec un écartement régulier entre eux et former un ensemble rotatif. Dans le cas de fixation par un bord seulement, une disposition avantageuse consiste à prévoir une fixation alternée en emboîtant successivement un bord longitudinal d'un plateau, puis le bord longitudinal opposé du plateau immédiatement voisin. L'agencement parallèle des plateaux s'avère de construction particulièrement simple.

Lorsque des cellules adhérentes sont cultivées dans l'enceinte, elles peuvent être recueillies à l'aide de moyens conventionnels tels qu'une solution d'enzymes protéolitiques comme la trypsine, le versène ou tout autre agent chélatant qui a pour but de détacher les cellules de leur support.

Le bioréacteur rotatif est conçu pour effectuer indifféremment des cultures de cellules adhérentes ou en suspension. Il peut également effectuer des cultures de cellules adhérentes en présence de microporteurs. Dans ce cas, toutes les surfaces intérieures prévues à cet effet et la surface constituée par les microporteurs peuvent être utilisées pour la croissance des cellules.

La rotation des plateaux d'un demi-tour dans un sens et puis dans l'autre permet de relier plusieurs appareils entre eux pendant leur fonctionnement. A cet effet, on prévoit avantageusement un repère d'orientation sur chaque appareil, par exemple sous la forme d'une marque, de manière à assurer un fonctionnement bien coordonné entre les différents appareils. Ceci permet, à l'aide de tuyaux comme mentionné plus haut, d'introduire ou d'évacuer des liquides à n'importe quel moment, de changer de milieu sans ouvrir l'enceinte et de prélever des échantillons représentatifs de microporteurs afin d'observer la croissance cellulaire.

L'appareil suivant l'invention permet d'effectuer avec un même flacon deux types de cultures, à savoir la culture de cellules ou micro-organismes adhérents ou la culture de cellules ou micro-organismes non adhérents, avec ou sans microporteurs. Cet avantage considérable et spécifique à l'invention permet ainsi de n'utiliser qu'un seul type de flacon, quel que soit le type de cellules ou de micro-organismes cultivés.

L'agencement de plusieurs plateaux permet d'augmenter considérablement la surface de culture à volume constant. Ainsi, l'appareil permet de produire une grande quantité de cellules dans un volume total restreint.

Il apparaît donc que l'appareil suivant l'invention permet d'accroître de manière considérable, dans un volume donné, à la fois la surface de culture et la surface d'échange entre les phases liquide et gazeuse. L'augmentation de surface par rapport à un flacon roulant ordinaire ou un fermenteur peut être supérieur à 1000 %.

Un autre avantage de l'appareil suivant l'invention est de maintenir les conditions de culture indépendamment de la taille de l'enceinte, le rapport entre le volume de liquide utilisé, le volume de la phase gazeuse et la surface totale de culture ou d'échange étant constant. Le passage d'une enceinte de petite capacité à une enceinte de plus grande capacité peut se faire dès lors sans modifier les paramètres régissant la culture.

L'appareil permet également de concentrer une phase liquide par évaporation. L'importante surface d'échange entre la phase gazeuse et la phase liquide permet, par circulation de gaz sec à l'intérieur de l'enceinte ou par aspiration de la phase gazeuse, d'évaporer partiellement ou intégralement la phase liquide contenue dans l'enceinte.

Les modes de réalisation de l'invention décrits dans ce qui précède sont des exemples donnés à titre illustratif et l'invention n'est nullement limitée à ces exemples. Toute modification, toute variante et tout agencement équivalent doivent être considérés comme compris dans le cadre de l'invention.

## Revendications

1. Appareil pour développer une surface d'échange réactionnel au sein d'un milieu liquide de réaction, l'appareil comprenant une enceinte fermée allongée (11) délimitant un volume pour recevoir un liquide de réaction et un volume pour une phase gazeuse, un dispositif pour introduire le liquide de réaction dans l'enceinte (11) et pour l'en évacuer, et au moins un ensemble de plateaux (12) disposés longitudinalement à l'intérieur de l'enceinte (11) avec un écartement entre eux,
caractérisé en ce que les plateaux (12) présentent le long de leurs bords, des rebords (31, 32, 33, 36) faisant saillie sur au moins une des faces opposées (24, 25) des plateaux de manière que, lorsque l'on imprime un mouvement de rotation à l'ensemble de plateaux précité, les plateaux (12) plongent dans le liquide de réaction et retiennent chacun une quantité prédéterminée de liquide pendant une fraction de leur course angulaire.

2. Appareil suivant la revendication 1, caractérisé en ce que les plateaux (12) sont fixés à l'enceinte extérieure (11) par au moins un de leurs bords longitudinaux (26, 27).

3. Appareil suivant la revendication 1, caractérisé en ce que les plateaux (12) sont fixés pratiquement radialement sur et autour d'un axe (20) s'étendant coaxialement à l'intérieur de l'enceinte.

4. Appareil suivant la revendication 3, caractérisé en ce que l'axe (20) précité est creux, la paroi dudit axe étant percée d'ouvertures (22).

5. Appareil suivant la revendication 4, caractérisé en ce que la surface intérieure de l'axe (20) creux présente des nervures longitudinales (23).

6. Appareil suivant l'une quelconque des revendications 3 à 5, caractérisé en ce que les plateaux (12) s'étendent depuis l'axe (20) jusqu'à l'enceinte extérieure (11).

7. Appareil suivant l'une quelconque des revendications 3 à 6, caractérisé en ce que, au voisinage de leur bord longitudinal libre, les plateaux (12) portent un rebord longitudinal (36) faisant saillie sur au moins une de ses faces (24, 25).

8. Appareil suivant la revendication 7, caractérisé en ce que chaque rebord longitudinal (36) porte au moins un organe distanceur (37) destiné à maintenir un écartement minimum prédéterminé entre les extrémités longitudinales libres des plateaux adjacents pour le passage de liquide.

9. Appareil suivant la revendication 6, caractérisé en ce que les plateaux (12) s'étendent jusqu'à l'enceinte extérieure (11) et sont également fixés à celle-ci.

10. Appareil suivant la revendication 1 ou 2, caractérisé en ce que les plateaux (12) sont montés de manière à s'étendre pratiquement parallèlement l'un à l'autre.

11. Appareil suivant la revendication 10, caractérisé en ce que les plateaux (12) sont fixés à l'enceinte extérieure (11) par leurs deux bords longitudinaux (26, 27) opposés.

12. Appareil suivant l'une quelconque des revendications précédentes, caractérisé en ce que les plateaux (12) sont fixés de manière amovible par logement d'au moins un bord longitudinal (26, 27) dans des rainures (14) formées dans la surface intérieure de l'enceinte.

13. Appareil suivant la revendication 12, caractérisé en ce que les rainures précitées ont une section transversale en forme de queue d'aronde.

14. Appareil suivant l'une quelconque des revendications précédentes, caractérisé en ce que les plateaux (12) présentent des ondulations sur pratiquement toute la surface d'une de leurs faces opposées (24, 25) au moins.

15. Appareil suivant l'une quelconque des revendications précédentes, caractérisé en ce que l'enceinte extérieure (11) est constituée d'un corps pratiquement cylindrique fermé à une extrémité par un fond (16) et à l'autre extrémité par une encolure (17) équipée d'un col (18) situé normalement sur l'axe longitudinal (100) de l'enceinte.

16. Appareil suivant la revendication 15, caractérisé en ce que le col (18) est fermé par un capot de fermeture (19).

17. Appareil suivant l'une quelconque des revendications 1 à 16, caractérisé en ce qu'il comprend en outre des moyens d'entraînement pour imprimer un mouvement de rotation à l'enceinte (11) et/ou à l'ensemble de plateaux (12).

18. Procédé pour développer une surface d'échange réactionnel au sein d'un milieu liquide de réaction contenu dans une enceinte, délimitant un volume pour recevoir un liquide de réaction et un volume pour une phase gazeuse, l'enceinte comprenant un ensemble de plateaux selon l'une quelconque des revendications 1 à 17, caractérisé en ce que l'on imprime à l'ensemble de plateaux (12) un mouvement de rotation autour d'un axe longitudinal de l'enceinte (11) alternativement d'une fraction de tour dans un sens, puis en sens inverse.

## Claims

1. An apparatus for developing a reactive exchange surface within a reaction liquid medium, the apparatus comprising a closed elongated housing (11) defining a volume to receive a reaction liquid and a volume for a gas phase, a device for introducing the reaction liquid into the housing (11) and for removing it therefrom, at least one set of trays (12) arranged longitudinally inside the housing (11) with a regular spacing between them, characterized in that the trays (12) have along their edges, rims (31, 32, 33, 36) which protrude from at least one of the opposite sides (24, 25) of the trays in such a way that, when the set of trays is subjected to a rotating movement, the trays (12) dip into the reaction liquid and each retains a predetermined quantity of liquid during a part of its angular travel.

2. The apparatus according to claim 1, wherein the trays (12) are attached to the housing (11) at least at one of their longitudinal edges (26, 27).

3. The apparatus according to claim 1, wherein the trays (12) are attached substantially radially to and around a shaft (20) extending coaxially inside the housing.

4. The apparatus according to claim 3, wherein the shaft (20) aforementioned is hollow, the wall of the said shaft being pierced with openings (22).

5. The apparatus according to claim 4, wherein the inner suface of the hollow shaft (20) has longitudinal ribs (23).

6. The apparatus according to either of claims 3 to 5, wherein the trays (12) extend from the shaft (20) to the enclosure (11).

7. The apparatus according to either of claims 3 to 6, wherein, next to their free longitudinal edge, the trays (12) have a longitudinal rim (36) which protrudes from at least one of its sides (24, 25).

8. The apparatus according to claim 7, wherein each longitudinal rim (36) carries at least one spacer element (37) destined to maintain a predetermined minimum spacing between the free longitudinal ends of the adjacent trays for allowing liquid to flow.

9. The apparatus according to claim 6, wherein the trays (12) extend as far as the housing (11) and are also attached to it.

10. The apparatus according to claim 1 or 2, wherein the trays (12) are mounted in such a way as to extend substantially parallel to one another.

11. The apparatus according to claim 10, wherein the trays (12) are attached to the housing (11) at their two opposite longitudinal edges (26, 27).

12. The apparatus according to any one of the preceding claims, wherein the trays (12) are removably attached to the housing inner surface by having at least one longitudinal edge (26, 27) fitted into grooves (14).

13. The apparatus according to claim 12, wherein the aforementioned grooves have a dovetail shaped transversal section.

14. The apparatus according to any one of the preceding claims, wherein the trays (12) have corrugations on substantially the whole surface of one of their opposite sides (24, 25) at least.

15. The apparatus according to any one of the preceding claims, wherein the housing (11) is comprised of a substantially cylindric body closed at one extremity by a base (16) and at the other extremity by a neck (17) equipped with a bottle neck (18) normally located on the longitudinal axis (100) of the housing.

16. The apparatus according to claim 15, wherein the bottle neck (18) is closed by a closing cap (19).

17. The apparatus according to any of the preceding claims, further comprising driving means to impart a rotating movement to the housing (11) and/or the set of trays (12).

18. A method of developing a reactive exchange surface within a reaction liquid medium contained in a housing having a longitudinal axis, said housing defining a volume to receive the reaction liquid and a volume for a gas phase and said housing comprising a set of trays according to any one of the preceding claims, characterized by subjecting the set of trays (12) to a rotating movement around said longitudinal axis of the housing (11) alternatively for a part of a turn in one direction and then in the opposite direction.

## Patentansprüche

1. Gerät zur Entwicklung einer reaktionellen Austauschfläche innerhalb eines flüssigen Reaktionsmedium, das Gerät bestehend aus einem geschlossenen, länglichen Gehäuse (11), das als Abgrenzung zwischen ein Volumen für eine Reaktionsflüssigkeit und ein Volumen für eine gasförmige Phase dient, einem Gerät zur Einführung der Flüssigkeit im Gehäuse und zu seiner Ableerung und mindestens einer Gruppe Schalen (12) der Länge nach im Gehäuse (11) installiert sind, dadurch gekennzeichnet, daß die Schalen (12) Ränder (31, 32, 33, 36) aufweisen, welche auf mindestens eine der gegenüberliegenden (24, 25) Schalenseiten überstehen, sodass die Schalen (12), während ihrer Rotation, in der Reaktionsflüssigkeit tauchen und jeweils eine vordefinierte Menge Flüssigkeit während eines Bruchteils ihrer winkelförmigen Laufbahn zurückhalten.

2. Gerät nach Anspruch 1, dadurch gekennzeichnet, daß die Schalen (12) durch mindestens eine ihrer Längskanten (26, 27) am aüsseren Gehäuse (11) befestigt sind.

3. Gerät nach Anspruch 1, dadurch gekennzeichnet, daß die Schalen (12) praktisch radial auf und rund um einer Achse, die Koaxial durch das Gehäuse läuft, befestigt sind.

4. Gerät nach Anspruch 3, dadurch gekennzeichnet, daß die obengenannte Achse (20) hohl und dessen Wand von Öffnungen durchbrochen ist (22).

5. Gerät nach Anspruch 4, dadurch gekennzeichnet, daß die innere Fläche der hohlen Achse (20) Längsrippen (23) vorzeigt.

6. Gerät nach einem der Ansprüche 3 bis 5, dadurch gekennzeichnet, daß die Schalen (12) von der Achse (20) bis zum aüsseren Gehäuse (11) reichen.

7. Gerät nach einem der Ansprüche 3 bis 6, dadurch gekennzeichnet, daß die Schalen (12) in der Nähe ihrer länglichen freien Kante einen länglichen Rand (36) haben der auf mindestens einer seiner Flächen übersteht (24, 25).

8. Gerät nach Anspruch 7, dadurch gekennzeichnet, daß jeder der länglichen Ränder (36) mindestens ein Distanzierungsorgan (37) hat, der dazu dient, eine vordefinierte Minimumentfernung zwischen den länglichen freien Rändern der aneindergrenzenden Schalen für die Passage der Flüssigkeit zu sichern.

9. Gerät nach Anspruch 6, dadurch gekennzeichnet, daß die Schalen (12) bis zum aüsseren Gehäuse (11) reichen und dass diese auch an ihm befestigt sind.

10. Gerät nach Anspruch 1 oder 2, dadurch gekennzeichnet, daß die Schalen so montiert sind, dass sie praktisch parallel zu einander liegen.

11. Gerät nach Anspruch 10, dadurch gekennzeichnet, daß die Schalen (12) durch ihre beiden länglichen (26, 27), gegenüberliegenden Kanten am aüsseren Gehäuse (11) befestigt sind.

12. Gerät nach einem der obengenanten Ansprüche, dadurch gekennzeichnet, daß die Schalen (12) herausnehmbar sind und durch mindestens eine längliche Kante (26, 27) in Rillen (14) auf die innere Fläche des Gehäuses stecken.

13. Gerät nach Anspruch 12, dadurch gekennzeichnet, daß die obengenannten Rillen einen schwalbenschwanzförmigen Querschnitt aufweisen.

14. Gerät nach einem der vorhergehenden Ansprüche, dadurch gekennzeichnet, daß die Schalen (12) praktisch auf der ganzen Fläche einer ihrer gegenüberliegenden Seiten (24, 25), Wellungen aufweisen.

15. Gerät nach einem der vorhergehenden Ansprüche, dadurch gekennzeichnet, daß das aüssere Gehäuse aus einem fast zylindrischen Gehäuse besteht, das an einem Ende von einem Boden (16) und am anderen von einem Halsauschnitt (17) mit einem Hals (18) der sich normalerweise auf die Längsachse (100) des Gehäuses befindet, abgeschlossen ist.

16. Gerät nach Anspruch 15, dadurch gekennzeichnet, daß der Hals (18) von einem Deckel (19) abgeschlossen wird.

17. Gerät nach einem der Ansprüche 1 bis 16, dadurch gekennzeichnet, daß es überdies mit Antriebsmitteln ausgerüstet ist um das Gehäuse (11) und/oder die Schalen (12) in eine Rotationsbewegung zu untersetzen.

18. Verfahren zur Entwicklung einer reaktionnellen Austauschfläche innerhalb eines flüssigen Reaktionsmediums das in einem Gehäuse enthalten ist das als Abgrenzung zwischen ein Volumen für eine Reaktionsflüssigkeit und ein Volumen für eine gasförmige Phase dient, wobei das Gehäuse Schalen nach einem der Ansprüche 1 bis 17 enthält die sich dadurch gekennzeichnet, daß man die Schalen (12) rund um eine Längsachse des Gehäuses (11), wechselweise um einen Bruchteil einer Rotation in einer Richtung und dann in die Gegenrichtung zum drehen bringt.
